# EUROPÄISCHE PATENTSCHRIFT

(11) **EP 4 449 994 B1**
(45) Veröffentlichungstag und Bekanntmachung des Hinweises auf die Patenterteilung: **29.10.2025**
(21) Anmeldenummer: 23200069.5
(22) Anmeldetag: 27.09.2023
(51) Int. Cl.: A61B 6/03, A61B 6/06, A61B 6/40, A61B 6/42, A61B 6/00

(54) **COMPUTERTOMOGRAPHIESYSTEM, DETEKTORRING UND VERFAHREN ZUM EINSTELLEN EINER BLENDENÖFFNUNG EINES COMPUTERTOMOGRAPHIESYSTEMS**
COMPUTED TOMOGRAPHY SYSTEM, DETECTOR RING AND METHOD FOR SETTING THE OPENING OF A COLLIMATOR OF A COMPUTER TOMOGRAPHY SYSTEM
SYSTÈME DE TOMOGRAPHIE ASSISTÉE PAR ORDINATEUR, BAGUE DE DÉTECTEUR ET PROCÉDÉ DE RÉGLAGE D'UNE OUVERTURE DE COLLIMATEUR D'UN SYSTÈME DE TOMOGRAPHIE ASSISTÉE PAR ORDINATEUR

(43) Veröffentlichungstag der Anmeldung: 23.10.2024
(73) Patentinhaber: Siemens Healthineers AG, 91301 Forchheim (DE)
(72) Erfinder: Mönius, Hannes, 91301 Forchheim (DE)
(74) Vertreter: Siemens Healthineers Patent Attorneys

(56) Entgegenhaltungen:
- CN-B- 102 764 137
- CN-U- 218 832 780
- US-A- 5 125 012
- US-A1- 2020 187 882
- CRAMER AVILASH ET AL: "Stationary Computed Tomography for Space and other Resource-constrained Environments", NATURE SCIENTIFIC REPORTS, vol. 8, no. 1, 21 September 2018 (2018-09-21), US, XP93150856, ISSN: 2045-2322, Retrieved from the Internet <URL:https://www.nature.com/articles/s41598-018-32505-z> DOI: 10.1038/s41598-018-32505-z

## Beschreibung

Die Erfindung betrifft ein Computertomographiesystem, einen Detektorring für ein Computertomographiesystem und ein Verfahren zum Einstellen einer Blendenöffnung eines Computertomographiesystems.

Unabhängig vom grammatikalischen Geschlecht eines bestimmten Begriffs sind Personen mit männlicher, weiblicher oder anderer Geschlechteridentität mit umfasst.

Übliche Computertomographiesysteme (CT-System) umfassen typischerweise eine Röntgenröhre und einen der Röntgenröhre gegenüberliegenden Detektor. Zur Bildgebung an einem Patienten rotieren Röntgenröhre und Detektor um den Patienten. Durch die Rotation kann ein dreidimensionales Bild des Patienten erfasst werden. Die Rotation bringt aber auch Nachteile mit sich. Aufgrund der Rotation sind die Anforderungen an die Mechanik und die Datenübertragung relativ groß. Diese erhöhten Anforderungen führen zu einem erhöhten Aufwand bei der Fertigung und Wartung sowie allgemein zu erhöhten Kosten und können auch die Bildqualität beeinträchtigen. Beispielsweise besteht das Risiko, dass durch die Rotation Vibrationen entstehen, welche störende Frequenzen in den Messsignalen und damit Artefakte in CT-Bildern verursachen können. Die Rotation im Allgemeinen und eventuelle durch die Rotation verursachte Vibrationen im Speziellen können zu einem schnelleren Verschleiß der Bauteile führen. Weiterhin bedingen die Rotationen, dass Bauteile besonders massiv gefertigt werden müssen, denn eine Verformung der Bauteile kann einen großen Einfluss auf die Bildqualität haben. Dies sorgt für einen erhöhten Platzbedarf und höhere Hardwarekosten. Auch ist durch die Rotation die Datenübertragung, beispielsweise über Schleifkontakte, erschwert, wodurch auch die Kapazität der Datenübertragung eingeschränkt sein kann.

Als Stand der Technik sind hierbei die US 5 125 012 A,
US 2020/187882 A1, CN 218 832 780 U, CN 102 764 137 B und Cramer Avilash et al.: "Stationary Computed Tomography for Space and other Resource-constrained Environments", Scientific Reports, Bd. 8, Nr. 1, 21. September 2018, XP93150856, zu nennen.

Es ist daher eine Aufgabe der vorliegenden Erfindung, eine Möglichkeit bereitzustellen, eine Blende in einem Computertomographiesystem mit einem Detektorring und einem Röntgenquellenring einstellen zu können.

Diese Aufgabe wird gelöst durch ein Computertomographiesystem gemäß einem der Ansprüche 1 bis 11 und ein Verfahren gemäß Anspruch 12. Weitere Merkmale und Vorteile ergeben sich aus den abhängigen Ansprüchen, der Beschreibung und den beigefügten Figuren.

Gemäß einem Aspekt der Erfindung ist ein Computertomographiesystem vorgesehen. Das Computertomographiesystem umfasst einen Untersuchungsbereich und eine statische Gantry. Die statische Gantry umfasst den erfindungsgemäßen Detektorring und den Röntgenquellenring, die jeweils den Untersuchungsbereich umschließen und im Wesentlichen eine gemeinsame Axialrichtung aufweisen. Der Röntgenquellenring weist den größeren Durchmesser auf als der Detektorring, sodass der Röntgenquellenring in Radialrichtung gesehen weiter außen um den Untersuchungsbereich angeordnet ist als der Detektorring. Der Detektorring ist in Axialrichtung so versetzt zu dem Röntgenquellenring angeordnet, dass eine Strahlaustrittsöffnung des Röntgenquellenrings in Radialrichtung gesehen zu dem Untersuchungsbereich hin zumindest teilweise nicht durch den Detektorring verdeckt ist.

Der Untersuchungsbereich ist insbesondere ein Bereich, in dem ein Subjekt oder Objekt mittels einer durch das Computertomographiesystem durchgeführten Computertomographiemessung untersucht werden kann, indem Computertomographie-(CT-)Bilder von dem Subjekt oder Objekt aufgenommen werden können. Das Subjekt oder Objekt kann beispielsweise ein Mensch oder ein Tier bzw. ein Körperteil, wie ein Organ oder ein Körperbereich, z.B. der Brustbereich oder Kopfbereich, oder ein anderes Objekt (z.B. Gepäckstücke oder Materialien, deren Eigenschaften untersucht werden sollen) sein.

Die Gantry, umfassend den Detektorring und den Röntgenquellenring, ist um den Untersuchungsbereich angeordnet. Die Gantry kann insbesondere ein kurzer Ringtunnel sein. Die Axialrichtung des Ringtunnels kann im Wesentlichen der Axialrichtung des Detektorrings und des Röntgenquellenrings entsprechen. Die Axialrichtung kann auch als z-Richtung bezeichnet werden. Der Begriff "im Wesentlichen" ist in diesem Zusammenhang so zu verstehen, dass auch gewisse, z.B. baulich bedingte, Abweichungen vorhanden sein können. Vorzugsweise weichen die Axialrichtungen der jeweils genannten Komponenten, insbesondere des Detektorrings und des Röntgenquellenrings, jedoch nicht um mehr als 10° voneinander ab. In diesem Kontext ist eine Radialrichtung so zu verstehen, dass sie senkrecht zu der Axialrichtung verläuft und insbesondere radial von einem Mittelpunkt der jeweiligen Ringe nach außen verläuft. Demgemäß ist der Begriff Radialrichtung so zu verstehen, dass es mehrere Richtungen gibt, die einer Radialrichtung entsprechen, nämlich gemäß der Gesamtheit der möglichen radial verlaufenden Richtungen. Die Radialrichtung kann auch als x-Richtung, y-Richtung und/oder x,y-Richtung bezeichnet werden.

Die Gantry ist statisch. Dies ist insbesondere so zu verstehen, dass, im Unterschied zu sonst üblichen Gantrys, keine Rotation der Gantry während einer CT-Messung vorgesehen ist. Dies wird dadurch ermöglicht, dass ein Detektorring und ein Röntgenquellenring vorgesehen sind. Somit kann auch ohne Rotation von Detektor und Röntgenquelle ein vollständiges Bild erzeugt werden. Insbesondere kann der Detektorring ein 360°-Detektor sein. Ein 360°-Detektor ist in diesem Sinne ein Detektor, welcher Signale, die von dem Untersuchungsbereich ausgehen in allen Radialrichtungen aufnehmen kann, ohne dabei selbst rotiert werden zu müssen. Insbesondere kann der Detektorring ein statischer Detektorring sein. Insbesondere kann der Röntgenquellenring ein 360°-Röntgenstrahler sein. Ein 360°-Röntgenstrahler ist in diesem Sinne ein Röntgenstrahler, welcher Röntgenstrahlen von über den gesamten 360°-Bereich um den Untersuchungsbereich in den Untersuchungsbereich senden kann, ohne dabei selbst rotiert werden zu müssen. Insbesondere kann der Röntgenquellenring ein statischer Röntgenquellenring sein. Der Detektorring kann beispielsweise mehrere Detektorelemente, welche um den Kreisumfang des Detektorrings verteilt sind, aufweisen. Der Röntgenquellenring kann beispielsweise mehrere Röntgenquellen, welche um den Kreisumfang des Röntgenquellenrings verteilt sind, aufweisen. Die Röntgenquellen können vorzugsweise auf einer Nanotube-Feldemissions-Technik basieren. Röntgenquellen, die auf Feldemission basieren, haben gegenüber in CT-Systemen des Standes der Technik üblicherweise verwendeten Röntgenquellen, die auf einer wärmebedingten Emission basieren, den Vorteil, dass ein schnelleres Umschalten ermöglicht werden kann, da ein Nachglüheffekt weitestgehend vermieden werden kann. Es können aber generell auch andere Röntgenquellen verwendet werden. Der Röntgenquellenring kann dazu konfiguriert sein, während eines CT-Scans eines Subjekts oder Objekts in dem Untersuchungsbereich einzelne oder Gruppen von mehreren seiner Röntgenquellen nacheinander zu aktivieren. Folglich kann eine Rotation bzw. der Vorteil einer Rotation gemäß den im Stand der Technik üblichen CT-Systemen quasi "simuliert" werden. Vorteilhafterweise kann somit eine tatsächliche Rotation der Gantry vermieden werden, womit die oben aufgelisteten Nachteile von rotierenden Teilen ebenfalls weitgehend vermieden werden können. Vorteilhafterweise sind somit keine in der Regel aufwendigen und teuren Schnittstellen zwischen relativ zueinander bewegten Teilen, wie zum Beispiel Schleifkontakte, nötig. Auch können rotationsbedingte Vibrationen und daraus resultierende Artefakte in den Messdaten und auftretender Verschleiß vermieden werden. Weiterhin ist eine kompaktere Bauweise möglich, weil weniger stabile Bauteile benötigt werden. Beispielsweise können Modulcarrier und eine Detektormechanik des Detektors bzw. des Detektorrings wesentlich einfacher, leichter und günstiger gebaut werden, da der Detektor keine Rotationskräfte aushalten muss. Beispielsweise kann es möglich sein, dass ein erfindungsgemäßes System auch für eine mobile Anwendung ausgestaltet ist. Weiterhin vorteilhafterweise kann es möglich sein, Bilder schneller aufzunehmen und flexibler in der Wahl aufeinanderfolgender Aufnahmewinkel zu sein, weil keine Trägheit bei mechanischer Rotation berücksichtigt werden muss.

Der Röntgenquellenring weist einen größeren Durchmesser auf als der Detektorring. Dadurch ist der Röntgenquellenring in Radialrichtung gesehen weiter außen um den Untersuchungsbereich angeordnet als der Detektorring. Mit anderen Worten ist der Röntgenquellenring weiter außen in der Gantry angeordnet als der Detektorring. Vorteilhafterweise können der Röntgenquellenring und der Detektorring dadurch besonders platzeffizient aufgebaut werden. Gleichzeitig ist der Detektorring in Axialrichtung so versetzt zu dem Röntgenquellenring angeordnet, dass die eine Strahlaustrittsöffnung des Röntgenquellenrings in Radialrichtung gesehen zu dem Untersuchungsbereich hin zumindest teilweise nicht, d.h. nicht vollständig, durch den Detektorring verdeckt ist. Insbesondere kann es vorgesehen sein, dass eine Mitte der Strahlaustrittsöffnung nicht durch den Detektorring verdeckt ist. Die Strahlaustrittsöffnung ist breit zu verstehen. Sie bezeichnet allgemein den Ort an dem Röntgenquellenring, an dem Röntgenstrahlen von den Röntgenquellen nach außen ausgesandt werden. In manchen Fällen, beispielsweise zum Zwecke einer Kollimation, insbesondere gemäß einigen hierin diskutierten Ausführungsbeispielen, kann es vorteilhaft sein, den Strahlenaustritt teilweise, insbesondere am Rand des Strahls, durch den durch Teile des Detektorrings oder Teile an dem Detektorring gezielt zu begrenzen. Der Detektorring kann optional als Kollimator oder Teil eines Kollimators für die Strahlaustrittsöffnung dienen und somit einen Teil der Strahlaustrittsöffnung verdecken. Es ist jedoch vorgesehen, dass zumindest ein Teil, vorzugsweise ein Zentrum, des Strahlaustrittsöffnung in der Regel nicht blockiert wird.

Gemäß einer bevorzugten Ausführungsform überlappen der Detektorring und der Röntgenquellenring in Axialrichtung teilweise. Durch diese Variante wird eine besonders kompakte Bauweise der Gantry ermöglicht. Weiterhin können der Detektorring und der Röntgenquellenring in Axialrichtung dadurch besonders eng zusammengebaut werden, wodurch eine verbesserte Bildqualität erzielt werden kann. Insbesondere kann somit ein Abstand von einem Fokuspunkt der Röntgenquellen des Röntgenquellenrings und der Detektormitte verringert werden. Dadurch können beispielsweise weniger ausgeprägte, durch einen Versatz in Axialrichtung verursachte, Bildfehler auftreten bzw. eine Bildkorrektur kann entsprechend erleichtert sein.

Gemäß einer Ausführungsform sind der Detektorring und der Röntgenquellenring in Axialrichtung gesehen von unterschiedlichen Seiten an der Gantry angebracht. Beispielsweise kann der Detektorring von positiver Axialrichtung (bzw. von +z-Richtung) an der Gantry montiert sein und der Röntgenquellenring kann von negativer Axialrichtung (bzw. von -z-Richtung) an der Gantry montiert sein. Durch die Montage von unterschiedlichen Seiten kann einerseits eine besonders gute Raumaufteilung möglich sein andererseits kann eine besonders gute Erreichbarkeit der beiden Komponenten, Röntgenquellenring und Detektorring, jeweils einzeln ermöglicht werden. Dies kann zum Beispiel für Wartungszwecke vorteilhaft sein. Beispielsweise kann der Detektorring abmontierbar oder teilweise abmontierbar sein, ohne dass der Röntgenquellenring die Demontage wesentlich erschwert. Beispielsweise kann der Detektorring somit direkt erreichbar und abmontierbar sein, wobei zu diesem Zwecke z.B. nur eine Abdeckplatte entfernt werden muss.

Gemäß einer Ausführungsform umfasst die Gantry die ringförmige Blende mit zumindest dem ersten Blendenteil und dem zweiten Blendenteil, wobei die Blende vor der Strahlaustrittsöffnung des Röntgenquellenrings angeordnet oder anordenbar ist, wobei das erste Blendenteil an dem Detektorring befestigt ist oder integraler Teil des Detektorrings ist. Insbesondere kann die Blende so vor der Strahlaustrittsöffnung angeordnet sein, dass die Blende die Röntgenstrahlen der Röntgenquellenrings kollimiert. Durch die Befestigung eines Blendenteils an dem Detektorring kann der Detektorring besonders nahe an der Strahlaustrittsöffnung angeordnet werden und es kann eine noch kompaktere Bauweise erzielt werden. Das zweite Blendenteil kann vorzugsweise an dem Röntgenquellenring oder einem anderen Teil der Gantry angebracht sein oder jeweils ein integraler Teil davon sein. Die Blende kann ein in Kreisumfangsrichtung des Röntgenquellenrings bzw. des Detektorrings komplett geschlossener Ring sein. Der erste Blendenteil und der zweite Blendenteil können gemeinsam einen Kanal bilden, welcher die Ausbreitung der Röntgenstrahlung begrenzt. Der Kanal kann insbesondere in Radialrichtung verlaufen. Der Kanal kann in Kreisumfangsrichtung gesehen einen im Wesentlichen konstanten Querschnitt und/oder eine konstante Kanalöffnung gegenüber der Strahlaustrittsöffnung aufweisen.

Gemäß einer Ausführungsform ist der Detektorring so konfiguriert, dass er zusammen mit dem ersten Blendenteil in Axialrichtung versetzbar ist, sodass durch ein Versetzen des Detektorrings in Axialrichtung eine Blendenöffnung der Blende einstellbar ist. Diese Ausführungsform kann eine effiziente Möglichkeit darstellen, um unterschiedliche Blendenöffnung für die Röntgenquellen zu realisieren. Insbesondere kann somit eine besonders platzsparende Möglichkeit einer einstellbaren Blende ermöglicht werden. Vorteilhafterweise kann somit gleichzeitig die Position des Detektorrings und die Strahlverteilung anpassbar sein. Damit kann es möglich sein, die Ausbreitung der Röntgenstrahlen und die Detektorposition einfach und effektiv zu synchronisieren.

Gemäß einer Ausführungsform umfasst das Computertomographiesystem weiterhin eine Patientenliege, wobei der Detektorring von der Seite der Patientenliege her in Axialrichtung an der Gantry angebracht ist. Die Patientenliege ist vorzugsweise in Axialrichtung in die Gantry ein- und ausfahrbar. Mit einer Anbringung des Detektorrings gemäß dieser Ausführungsform kann eine besonders einfache Zugänglichkeit zu dem Detektorring, beispielsweise zu Wartungszwecken, ermöglicht werden. Insbesondere kann der Detektorring oder können Elemente des Detektorrings besonders einfach aus der Gantry entnehmbar sein.

Gemäß einer Ausführungsform sind die Funktionseinheiten einzeln von der Gantry abnehmbar. Vorteilhafterweise kann bei einzeln entnehmbaren Funktionseinheiten beispielsweise eine Wartung oder Ersetzung von einzelnen Detektorelementen möglich sein, ohne den gesamten Detektorring entnehmen zu müssen. Beispielsweise kann es somit einfacher möglich sein, eine generelle Ausrichtung und Justierung des Detektorrings beizubehalten, sodass nur einzelnen Funktionseinheiten wieder installiert werden müssen, während die generelle Ausrichtung des Detektorrings (und ggf. der Blende) unverändert bleiben kann. Dies kann eine Wartung vereinfachen und kostengünstiger machen. Der Detektorring kann optional weitere Funktionseinheiten umfassen. Einzelne Funktionseinheiten können Module, Kabel und/oder DSF (Data Streaming Frontend) umfassen oder sein. Beispielsweise kann eine Funktionseinheit ein Modul mit einer teilkreisförmigen Anordnung von Detektorelementen sein.

Gemäß einer Ausführungsform umfasst der Detektorring zumindest einen Gehäuseabschnitt, wobei der Gehäuseabschnitt so ausgestaltet ist, dass zumindest eine der Funktionseinheiten zumindest teilweise in den Gehäuseabschnitt einsetzbar ist. Zumindest eine Funktionseinheit kann in dem Gehäuseabschnitt eingesetzt sein. Beispielsweise kann es vorgesehen sein, dass die zumindest eine Funktionseinheit während eines Betriebs des Detektorrings in dem Gehäuseabschnitt eingesetzt ist. Es kann vorgesehen sein, dass die Funktionseinheit für eine Wartung der Funktionseinheit aus dem Gehäuse entnommen wird oder entnehmbar ist. Vorzugsweise kann der Gehäuseabschnitt ringförmig oder teilringförmig sein. Der Gehäuseabschnitt kann insbesondere so ausgestaltet sein, dass die Detektorelemente zumindest einer Funktionseinheit in den Gehäuseabschnitt eingesetzt werden können. Vorteilhafterweise können die Detektorelemente somit eine durch die Position des Gehäuseabschnitts definierte Position haben. Somit kann es möglich sein, Detektorelemente auszutauschen, ohne anschließend deren Position erneut einstellen zu müssen oder es kann vereinfacht möglich sein, die Position der Detektorelemente einzustellen. Es kann vorgesehen sein, dass mehrere Funktionseinheiten mit Detektorelementen in einem Gehäuseabschnitt einsetzbar oder eingesetzt sind. Beispielsweise kann ein Gehäuseabschnitt so ausgestaltet sein, dass jeweils zwei der Funktionseinheiten zumindest teilweise in den Gehäuseabschnitt einsetzbar sind. Eine Einsetzbarkeit von jeweils zwei Funktionseinheiten kann besonders vorteilhaft sein, um gleichermaßen eine stabile Positionierung als auch eine einfache Wartung zu ermöglichen. Das erste Blendenteil wie hierin beschrieben kann vorzugsweise an dem Gehäuseabschnitt befestigt sein. Vorzugsweise kann somit verhindert werden, dass eine Veränderung der Position des ersten Blendenteils bei der Entnahme oder dem Austausch der Funktionseinheit erfolgt.

Gemäß einer Ausführungsform umfasst der Detektorring mehrere Gehäuseabschnitte, die zusammengesetzt die Ringform des Detektorrings definieren. In diesem Sinne können die Gehäuseabschnitte Segmente, insbesondere Kreissegmente, des Detektorrings sein. Die Gehäuseabschnitte können im Wesentlichen gleich große Kreissegmente des Detektorrings sein. Bevorzugt kann der Detektorring drei oder mehr Gehäuseabschnitte umfassen. Besonders bevorzugt kann der Detektorring drei Gehäuseabschnitte umfassen. Die drei Gehäuseabschnitte können insbesondere im Wesentlichen drei gleich große Kreissegmente des Detektorrings sein. Beispielsweise können die Gehäuseabschnitte jeweils 120°-Segmente sein. Eine Aufteilung in drei Gehäuseabschnitte hat sich als besonders vorteilhaft für Wartungszwecke und eine flexible Anpassung der Ausrichtung des Detektorrings herausgestellt. Das erste Blendenteil wie hierin beschrieben kann mehrere erste Blendenteile umfassen bzw. aus mehreren ersten Blendenteilen zusammengesetzt sein, welche jeweils an einem der Gehäuseabschnitte befestigt sind.

Gemäß einer Ausführungsform umfasst zumindest eine der Funktionseinheiten eine Führung, die dazu ausgerichtet ist, ein ortspräzises Einsetzen der Funktionseinheit in dem Gehäuseabschnitt zu ermöglichen. Alternativ oder zusätzlich kann die Führung als Teil des Gehäuseabschnitts vorgesehen sein.
Die Führung kann beispielsweise eine Führungsschiene oder ein Führungsstab sein. Der Gehäuseabschnitt kann beispielsweise eine Innenkante oder einen Führungstunnel umfassen, der dazu ausgestaltet ist, mit der Führung der Funktionseinheit zusammenzuwirken. Mit der Führung kann eine besonders präzise Positionierung der Funktionseinheit in dem Gehäuseabschnitt ermöglicht werden. Weiterhin kann die Führung dazu beitragen, dass eine Beschädigung der Funktionseinheit, insbesondere der Detektorelemente, z.B. durch Stöße beim Einsetzen, verhindert werden kann.

Gemäß einer Ausführungsform umfasst der Detektorring und/oder der Gehäuseabschnitt einen Streustrahlkollimator, der dazu ausgerichtet ist, Detektorelemente des Detektorrings vor Streustrahlen des Röntgenquellenrings abzuschirmen, wobei der Gehäuseabschnitt so ausgestaltet ist, dass bei einem Einsetzen der zumindest einen Funktionseinheit in den Gehäuseabschnitt die Detektorelemente der zumindest einen Funktionseinheit passgenau zu dem Streustahlkollimator eingesetzt werden. Vorteilhafterweise kann somit eine Positionierung des Streustrahlkollimators beibehalten werden, während beispielsweise eine Funktionseinheit ausgetauscht wird.

Gemäß einer Ausführungsform umfasst der Röntgenquellenring mehrere Röntgenquellensegmente. Alle Vorteile und Merkmale betreffend die Wartung des Detektorrings können analog übertragen werden auf den Röntgenquellenring. Somit können vorteilhafterweise einzelne Segmente des Röntgenquellenrings entnehmbar sein. Beispielsweise können auch entsprechende Funktionseinheiten und/oder Gehäuseabschnitte des Röntgenquellenrings vorgesehen sein.

Ein weiterer Aspekt der Erfindung ist ein Detektorring für ein Computertomographiesystem, wobei der Detektorring zumindest einen ringförmigen oder teilringförmigen Gehäuseabschnitt und eine oder mehrere Funktionseinheiten mit Detektorelementen umfasst, wobei der Gehäuseabschnitt so ausgestaltet ist, dass zumindest eine der Funktionseinheiten zumindest teilweise in den Gehäuseabschnitt einsetzbar oder eingesetzt ist. Der Detektorring kann insbesondere ein Detektorring wie hierin mit Bezug auf das Computertomographiesystem beschrieben sein. Alle Vorteile und Merkmale des Computertomographiesystems können analog auf den Detektorring übertragen werden und umgekehrt. Der Detektorring kann mehrere Gehäuseabschnitte wie hierin beschrieben umfassen, die zusammengesetzt die Ringform des Detektorrings definieren.

Gemäß einer Ausführungsform umfasst zumindest eine der Funktionseinheiten eine Führung, die dazu ausgerichtet ist, ein ortspräzises Einsetzen der Funktionseinheit in dem Gehäuseabschnitt zu ermöglichen. Alternativ oder zusätzlich kann die Führung als Teil des Gehäuseabschnitts vorgesehen sein. Die Führung kann beispielsweise eine Führungsschiene oder ein Führungsstab sein. Der Gehäuseabschnitt kann beispielsweise eine Innenkante oder einen Führungstunnel umfassen, der dazu ausgestaltet ist, mit der Führung der Funktionseinheit zusammenzuwirken.

Gemäß der Erfindung umfasst der Detektorring ein erstes Blendenteil, das an dem Detektorring befestigt ist oder integraler Teil des Detektorrings ist, wobei das erste Blendenteil dazu ausgestaltet ist zusammen mit einem zweiten Blendenteil eine Blende für einen Röntgenquellenring, der einen größeren Durchmesser aufweist als der Detektorring, zu ergeben. Insbesondere kann die Blende für einen Röntgenquellenring wie hierin beschrieben vorgesehen sein. Das erste Blendenteil kann beispielsweise an dem Gehäuseabschnitt befestigt sein.
Gemäß einer Ausführungsform umfasst der Detektorring und/oder der Gehäuseabschnitt einen Streustrahlkollimator, wobei der Gehäuseabschnitt so ausgestaltet ist, dass bei einem Einsetzen der zumindest einen Funktionseinheit in den Gehäuseabschnitt die Detektorelemente der zumindest einen Funktionseinheit passgenau zu dem Streustahlkollimator eingesetzt werden.

Ein weiterer Aspekt der Erfindung ist ein Verfahren zum Einstellen einer Blendenöffnung eines Computertomographiesystems mit statischer Gantry, wobei die Gantry einen Detektorring und einen Röntgenquellenring mit einer Strahlaustrittsöffnung sowie eine ringförmige Blende mit zumindest einem ersten Blendenteil und einem zweiten Blendenteil umfasst, wobei die Blende vor der Strahlaustrittsöffnung des Röntgenquellenrings, der einen größeren Durchmesser aufweist als der Detektorring, angeordnet ist, wobei der Detektorring zumindest einen ringförmigen oder teilringförmigen Gehäuseabschnitt und eine oder mehrere Funktionseinheiten mit Detektorelementen umfasst, wobei der Gehäuseabschnitt so ausgestaltet ist, dass zumindest eine der Funktionseinheiten zumindest teilweise in den Gehäuseabschnitt einsetzbar ist, wobei das erste Blendenteil an dem Detektorring befestigt ist oder integraler Teil des Detektorrings ist. Das Computertomographiesystem kann insbesondere ein Computertomographiesystems wie hierin beschrieben sein. Das Verfahren umfasst den folgenden Schritt: - Versetzen des Detektorrings und damit des ersten Blendenteils in Axialrichtung, sodass ein Abstand des ersten Blendenteils zu dem zweiten Blendenteil verändert wird und die Blendenöffnung der Blende eingestellt wird.
Alle Vorteile und Merkmale des Computertomographiesystems und des Detektorrings können analog auf das Verfahren übertragen werden und umgekehrt.

Alle hierin beschriebenen Ausführungsformen können miteinander kombiniert werden, soweit nicht explizit etwas anderes angegeben ist.

Im Folgenden werden Ausführungsformen mit Bezug auf die beigefügten Figuren beschrieben.
- Fig. 1: zeigt eine schematische Schnittansicht von der Seite auf ein Computertomographiesystem gemäß einer Ausführungsform der Erfindung,
- Fig. 2: zeigt eine Perspektivansicht auf eine Gantry eines Computertomographiesystems gemäß einer Ausführungsform der Erfindung,
- Fig. 3: zeigt einen Gehäuseabschnitt und zwei Funktionseinheiten eines Detektorrings gemäß einer Ausführungsform der Erfindung und
- Fig. 4: zeigt eine schematische Darstellung eines Verfahrens zum Einstellen einer Blendenöffnung eines Computertomographiesystems mit statischer Gantry gemäß einer Ausführungsform der Erfindung.

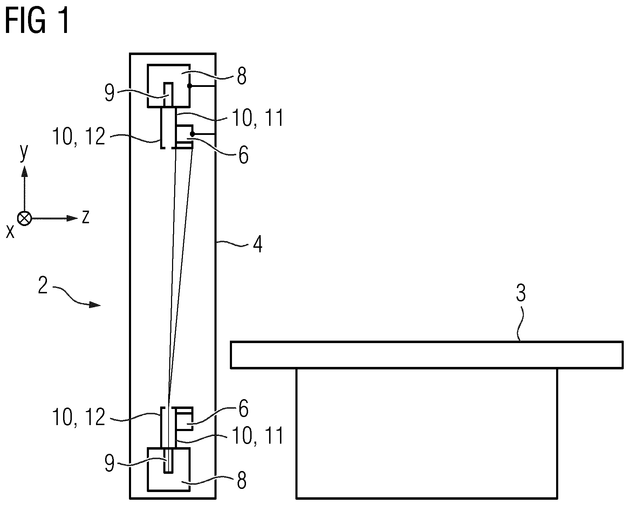

Figur 1 zeigt eine schematische Schnittansicht von der Seite auf ein Computertomographiesystem gemäß einer Ausführungsform der Erfindung. Das Computertomographiesystem umfasst eine statische Gantry 4, welche als kurzer Ringtunnel um einen Untersuchungsbereich 2 angeordnet ist. Die Gantry 4 umfasst einen Detektorring 6 und einen Röntgenquellenring 8, von denen jeweils ein oberer Teil und ein unterer Teil eingezeichnet ist und die jeweils den Untersuchungsbereich 2 umschließen. Detektorring 6 und Röntgenquellenring 8 weisen eine gemeinsame Axialrichtung auf, welche der eingezeichneten z-Richtung entspricht. Der Detektorring 6 ist, bezogen auf diese Darstellung, von der rechten Seite und der Röntgenquellenring 8 ist von der linken Seite an der Gantry 4 angebracht. Das bedeutet, Detektorring 6 und Röntgenquellenring 8 sind von verschiedenen Seiten an der Gantry 4 angebracht. Dabei ist der Detektorring 6 von der Seite an der Gantry 4 angebracht, auf der auch eine Patientenliege 3 vorgesehen ist. Der Detektorring 6 und der Röntgenquellenring 8 überlappen in Axialrichtung teilweise, d.h. ein Teil des Röntgenquellenrings 8 ist an der gleichen z-Position wie ein Teil des Detektorrings 6.

Der Röntgenquellenring 8 weist einen größeren Durchmesser auf als der Detektorring 6, sodass der Röntgenquellenring 8 in Radialrichtung gesehen, also in x,y-Richtung, weiter außen um den Untersuchungsbereich 2 angeordnet ist als der Detektorring 6. Zudem ist der Detektorring 6 in Axialrichtung so versetzt zu dem Röntgenquellenring 8 angeordnet, dass eine Strahlaustrittsöffnung 9 des Röntgenquellenrings 8 in Radialrichtung gesehen zu dem Untersuchungsbereich 2 hin nicht vollständig durch den Detektorring 6 verdeckt ist. An dem Detektorring 6 ist ein erstes Blendenteil 11 befestigt, das Teil einer ringförmigen Blende 10 ist, die vor der Strahlaustrittsöffnung 9 angeordnet ist. Ein zweites Blendenteil 12 der Blende 10 ist an dem Röntgenquellenring 8 selbst befestigt. In dieser Ausführungsform und vorzugsweise auch in anderen Ausführungsformen, sind Blendenteil 11 und Blendenteil 12 insbesondere unabhängige Baugruppen, die jedoch funktionstechnisch zusammenspielen. Der Detektorring 6 ist so ausgestaltet, dass er zusammen mit dem ersten Blendenteil 11 in Axialrichtung (d.h. hier z-Richtung) versetzbar ist, sodass durch ein Versetzen des Detektorrings 6 in Axialrichtung eine Blendenöffnung der Blende 10 einstellbar ist.

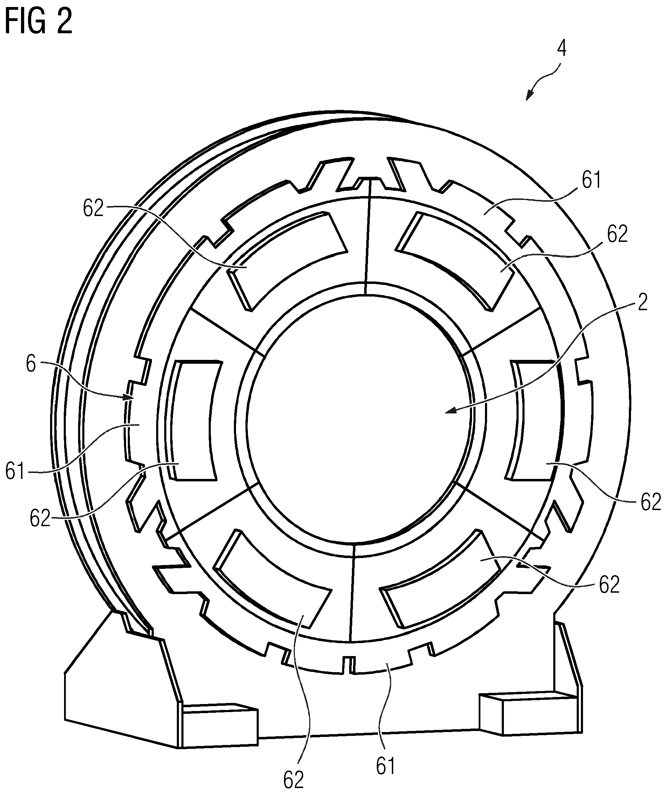

Figur 2 zeigt eine Perspektivansicht auf eine Gantry 4 eines Computertomographiesystems gemäß einer Ausführungsform der Erfindung. In diesem Fall ist die Gantry 4 ohne äußere Verkleidung dargestellt. An der Gantry 4 ist ein Detektorring 6 angebracht. Auf der gegenüberliegenden Seite der Gantry 4 (aus dieser Perspektive die Rückseite) ist ein Röntgenquellenring 8 vorgesehen (hier nicht zu sehen). Der Detektorring 6 umfasst drei teilringförmige Gehäuseabschnitte 61 sowie, für jeden Gehäuseabschnitt 61 jeweils zwei Funktionseinheiten 62 mit Detektorelementen 63, die in dem Gehäuseabschnitt 61 eingesetzt sind. Die Gehäuseabschnitte 61 ergeben zusammen die Ringform des Detektorrings 6. Die Funktionseinheiten 62 sind jeweils einzeln von der Gantry 4 abnehmbar. Der hier nicht sichtbare Röntgenquellenring 8 kann optional ebenfalls mehrere Röntgenquellensegmente umfassen, analog zu den Funktionseinheiten 62 bzw. Gehäuseabschnitten 61 des Detektorrings 6.

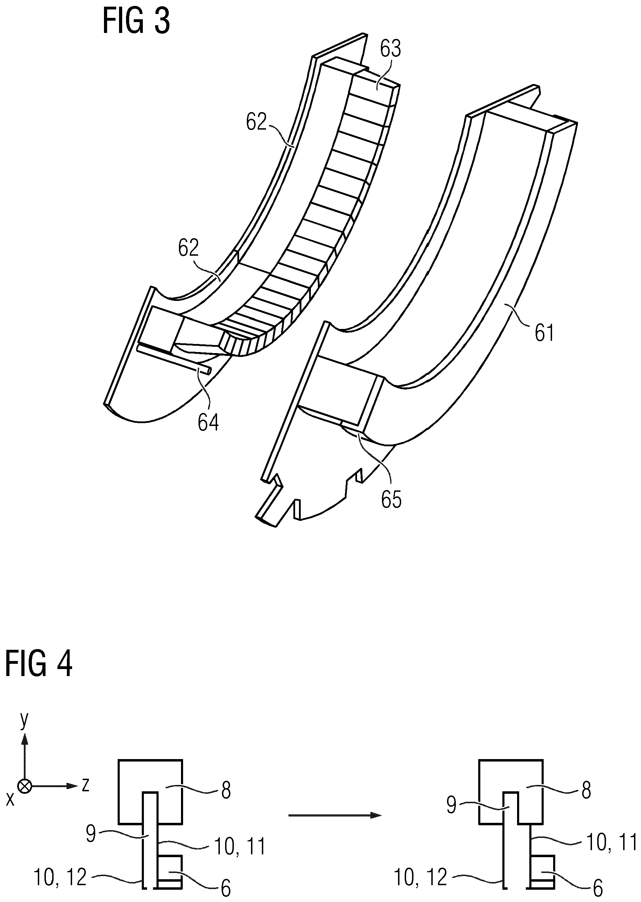

Figur 3 zeigt einen Gehäuseabschnitt 61 und zwei Funktionseinheiten 62 eines Detektorrings 6 gemäß einer Ausführungsform der Erfindung. Die Detektorelemente 63 der beiden Funktionseinheiten 62 sind in die Gehäuseabschnitt 61 einsetzbar. Die Funktionseinheiten 62 umfassen jeweils eine Führung 64 in Form eines Führungsstabs, die dazu ausgerichtet ist, ein ortspräzises Einsetzen der Funktionseinheiten 62 in dem Gehäuseabschnitt 61 zu ermöglichen, indem die Führung 64 an einer Innenkante des Gehäuseabschnitts 61 entlanggeführt wird und somit eine Ausrichtung der jeweiligen Funktionseinheit 62 bewirkt. Der Gehäuseabschnitt 61 umfasst weiterhin einen Streustrahlkollimator 65, der dazu ausgerichtet ist, die Detektorelemente 63 vor Streustrahlen des Röntgenquellenrings 8 abzuschirmen. Durch die Führung 64 kann weiterhin gewährleistet werden, dass bei einem Einsetzen der Funktionseinheiten 62 in den Gehäuseabschnitt 61 die Detektorelemente 63 passgenau zu dem Streustahlkollimator 65 eingesetzt werden.

Figur 4 zeigt eine schematische Darstellung eines Verfahrens zum Einstellen einer Blendenöffnung eines Computertomographiesystems mit statischer Gantry 4 gemäß einer Ausführungsform der Erfindung. Die Gantry 4 umfasst einen Detektorring 6 und einen Röntgenquellenring 8 mit einer Strahlaustrittsöffnung 9 sowie eine ringförmige Blende 10 mit einem ersten Blendenteil 11 und einem zweiten Blendenteil 12, die vor der Strahlaustrittsöffnung 9 des Röntgenquellenrings 8 angeordnet ist. Das erste Blendenteil 11 ist an dem Detektorring 6 befestigt. Wie angedeutet wird bei dem Verfahren des Detektorring 6 und damit auch das erste Blendenteil 11 in Axialrichtung (z-Richtung) versetz, sodass ein Abstand des ersten Blendenteils 11 zu dem zweiten Blendenteil 12 verändert (in diesem Fall vergrößert) wird und die Blendenöffnung der Blende 10 eingestellt wird.

## Patentansprüche

1. Computertomographiesystem umfassend einen Untersuchungsbereich (2) und eine statische Gantry (4) umfassend einen Detektorring (6) und einen Röntgenquellenring (8), die jeweils den Untersuchungsbereich (2) umschließen und im Wesentlichen eine gemeinsame Axialrichtung aufweisen, wobei der Röntgenquellenring (8) einen größeren Durchmesser aufweist als der Detektorring (6), sodass der Röntgenquellenring (8) in Radialrichtung gesehen weiter außen um den Untersuchungsbereich (2) angeordnet ist als der Detektorring (6), wobei der Detektorring (6) in Axialrichtung so versetzt zu dem Röntgenquellenring (8) angeordnet ist, dass eine Strahlaustrittsöffnung (9) des Röntgenquellenrings (8) in Radialrichtung gesehen zu dem Untersuchungsbereich (2) hin zumindest teilweise nicht durch den Detektorring (6) verdeckt ist,
**dadurch gekennzeichnet, dass**
der Detektorring zumindest einen ringförmigen oder teilringförmigen Gehäuseabschnitt (61) und eine oder mehrere Funktionseinheiten (62) mit Detektorelementen (63) umfasst, wobei der Gehäuseabschnitt (61) so ausgestaltet ist, dass zumindest eine der Funktionseinheiten (62) zumindest teilweise in den Gehäuseabschnitt (61) einsetzbar ist, und dass der Detektorring (6) ein erstes Blendenteil (11) umfasst, das an dem Detektorring (6) befestigt ist oder integraler Teil des Detektorrings (6) ist, wobei das erste Blendenteil (11) dazu ausgestaltet ist zusammen mit einem zweiten Blendenteil (12) eine Blende (10) für den Röntgenquellenring (8) zu ergeben.

2. Computertomographiesystem gemäß Anspruch 1, wobei der Detektorring (6) und der Röntgenquellenring (8) in Axialrichtung gesehen von unterschiedlichen Seiten an der Gantry (4) angebracht sind.

3. Computertomographiesystem gemäß Anspruch 1 oder 2,
wobei die Gantry (4) die ringförmige Blende (10) mit zumindest dem ersten Blendenteil (11) und dem zweiten Blendenteil (12) umfasst,
wobei die Blende vor der Strahlaustrittsöffnung (9) des Röntgenquellenrings (8) angeordnet ist.

4. Computertomographiesystem gemäß Anspruch 3, wobei der Detektorring (6) so ausgestaltet ist, dass er zusammen mit dem ersten Blendenteil (11) in Axialrichtung versetzbar ist, sodass durch ein Versetzen des Detektorrings (6) in Axialrichtung eine Blendenöffnung der Blende (10) einstellbar ist.

5. Computertomographiesystem gemäß Anspruch 3 oder 4, wobei das zweite Blendenteil (12) an dem Röntgenquellenring (8) oder einem anderen Teil der Gantry (4) angebracht ist oder jeweils ein integraler Teil davon ist.

6. Computertomographiesystem gemäß einem der Ansprüche bis 5, wobei der Detektorring (6) und der Röntgenquellenring (8) in Axialrichtung teilweise überlappen.

7. Computertomographiesystem gemäß einem der Ansprüche 1 bis 6,
wobei das Computertomographiesystem weiterhin eine Patientenliege (3) umfasst, die insbesondere in Axialrichtung in die Gantry (4) ein- und ausfahrbar ist,
wobei der Detektorring (6) von der Seite der Patientenliege (3) her in Axialrichtung an der Gantry (4) angebracht ist.

8. Computertomographiesystem gemäß einem der Ansprüche 1 bis 7, wobei die Funktionseinheiten (62) einzeln von der Gantry (4) abnehmbar sind.

9. Computertomographiesystem gemäß Anspruch 8, wobei zumindest eine der Funktionseinheiten (62) eine Führung (64) umfasst, die dazu ausgerichtet ist, ein ortspräzises Einsetzen der Funktionseinheit (62) in dem Gehäuseabschnitt (61) zu ermöglichen.

10. Computertomographiesystem gemäß Anspruch 8 oder 9,
wobei der Gehäuseabschnitt (61) einen Streustrahlkollimator (65) umfasst, der dazu ausgerichtet ist, Detektorelemente (63) des Detektorrings (6) vor Streustrahlen des Röntgenquellenrings (8) abzuschirmen,
wobei der Gehäuseabschnitt (61) so ausgestaltet ist, dass bei einem Einsetzen der zumindest einen Funktionseinheit (62) in den Gehäuseabschnitt (61) die Detektorelemente (63) der zumindest einen Funktionseinheit (62) passgenau zu dem Streustahlkollimator eingesetzt werden.

11. Computertomographiesystem gemäß einem der Ansprüche 8 bis 10, wobei der Detektorring (6) mehrere Gehäuseabschnitte (61) umfasst, die zusammengesetzt die Ringform des Detektorrings (6) definieren.

12. Verfahren zum Einstellen einer Blendenöffnung eines Computertomographiesystems mit statischer Gantry (4), insbesondere eines Computertomographiesystems gemäß einem der Ansprüche 1 bis 11,
wobei die Gantry (4) einen Detektorring (6) und einen Röntgenquellenring (8) mit einer Strahlaustrittsöffnung (9) sowie eine ringförmige Blende (10) mit zumindest einem ersten Blendenteil (11) und einem zweiten Blendenteil (12) umfasst,
wobei die Blende (10) vor der Strahlaustrittsöffnung (9) des Röntgenquellenrings (8), der einen größeren Durchmesser aufweist als der Detektorring (6), angeordnet ist,
wobei der Detektorring (6) zumindest einen ringförmigen oder teilringförmigen Gehäuseabschnitt (61) und eine oder mehrere Funktionseinheiten (62) mit Detektorelementen (63) umfasst,
wobei der Gehäuseabschnitt (61) so ausgestaltet ist, dass zumindest eine der Funktionseinheiten (62) zumindest teilweise in den Gehäuseabschnitt (61) einsetzbar ist,
wobei das erste Blendenteil an dem Detektorring (6) befestigt ist oder integraler Teil des Detektorrings (6) ist,
wobei das Verfahren den folgenden Schritt umfasst:
- Versetzen des Detektorrings (6) und damit des ersten Blendenteils (11) in Axialrichtung, sodass ein Abstand des ersten Blendenteils (11) zu dem zweiten Blendenteil (12) verändert wird und die Blendenöffnung der Blende (10) eingestellt wird.

## Claims

1. Computed tomography system comprising
an examination region (2) and
a static gantry (4) comprising a detector ring (6) and an X-ray source ring (8), which each enclose the examination region (2) and essentially have a common axial direction, wherein the X-ray source ring (8) has a larger diameter than the detector ring (6), so that the X-ray source ring (8), when viewed in the radial direction, is arranged further out around the examination region (2) than the detector ring (6),
wherein the detector ring (6) is arranged offset in the axial direction with respect to the X-ray source ring (8) in such a manner that a beam outlet opening (9) of the X-ray source ring (8), when viewed in the radial direction with respect to the examination region (2), is at least in part not covered by the detector ring (6),
**characterised in that**
the detector ring (6) comprises at least one annular or partially annular housing section (61) and one or multiple functional units (62) having detector elements (63), wherein the housing section (61) is configured in such a manner that at least one of the functional units (62) can be inserted at least in part into the housing section (61), and that
the detector ring (6) comprises a first aperture part (11), which is fastened to the detector ring (6) or is an integral part of the detector ring (6), wherein the first aperture part (11) is configured so as to produce, together with a second aperture part (12), an aperture (10) for the X-ray emission ring (8).

2. Computed tomography system according to claim 1,
wherein the detector ring (6) and the X-ray source ring (8) are attached to the gantry (4) from different sides, when viewed in the axial direction.

3. Computed tomography system according to claim 1 or 2,
wherein the gantry (4) comprises the annular aperture (10) having at least the first aperture part (11) and the second aperture part (12),
wherein the aperture is arranged in front of the beam outlet opening (9) of the X-ray source ring (8).

4. Computed tomography system according to claim 3,
wherein the detector ring (6) is configured in such a manner that it can be displaced in the axial direction together with the first aperture part (11), so that an aperture opening of the aperture (10) can be adjusted by displacing the detector ring (6) in the axial direction.

5. Computed tomography system according to claim 3 or 4,
wherein the second aperture part (12) is attached to the X-ray source ring (8) or to another part of the gantry (4) or is an integral part thereof in each case.

6. Computed tomography system according to one of claims 1 to 5,
wherein the detector ring (6) and the X-ray source ring (8) partially overlap in the axial direction.

7. Computed tomography system according to one of claims 1 to 6,
wherein the computed tomography system further comprises a patient couch (3), which can be moved into and out of the gantry (4), in particular in the axial direction,
wherein the detector ring (6) is attached to the gantry (4) from the side of the patient couch (3) in the axial direction.

8. Computed tomography system according to one of claims 1 to 7,
wherein the functional units (62) can be removed individually from the gantry (4).

9. Computed tomography system according to claim 8,
wherein at least one of the functional units (62) comprises a guide (64) that is oriented so as to enable the functional unit (62) to be inserted in the housing section (61) in a precise manner in terms of positioning.

10. Computed tomography system according to claim 8 or 9,
wherein the housing section (61) comprises a scattered beam collimator (65), which is oriented to shield detector elements (63) of the detector ring (6) from scattered beams of the X-ray source ring (8),
wherein the housing section (61) is configured in such a manner that when the at least one functional unit (62) is inserted into the housing section (61), the detector elements (63) of the at least one functional unit (62) are inserted in a precisely fitting manner with respect to the scattered beam collimator.

11. Computed tomography system according to one of claims 8 to 10,
wherein the detector ring (6) comprises multiple housing sections (61), which, when assembled, define the annular shape of the detector ring (6).

12. Method for adjusting an aperture opening of a computed tomography system having a static gantry (4), in particular a computed tomography system according to one of claims 1 to 11,
wherein the gantry (4) comprises a detector ring (6) and an X-ray source ring (8) having a beam outlet opening (9) and an annular aperture (10) having at least a first aperture part (11) and a second aperture part (12),
wherein the aperture (10) is arranged in front of the beam outlet opening (9) of the X-ray source ring (8), which has a larger diameter than the detector ring (6),
wherein the detector ring (6) comprises at least one annular or partially annular housing section (61) and one or multiple functional units (62) having detector elements (63), wherein the housing section (61) is configured in such a manner that at least one of the functional units (62) can be inserted at least in part into the housing section (61),
wherein the first aperture part is fastened to the detector ring (6) or is an integral part of the detector ring (6),
wherein the method comprises the following step:
- displacing the detector ring (6) and thus the first aperture part (11) in the axial direction, so that a distance between the first aperture part (11) and the second aperture part (12) is changed and the aperture opening of the aperture (10) is adjusted.

## Revendications

1. Système de tomodensitométrie assisté par ordinateur comprenant
une partie (2) d'examen et
un portique (4) statique comprenant un anneau (6) formant détecteur et un anneau (8) formant source de rayons X, qui entourent chacun la partie (2) d'examen et ont essentiellement une direction axiale commune,
dans lequel l'anneau (8) formant source de rayons X a un diamètre plus grand que l'anneau (6) formant détecteur, de sorte que l'anneau (8) formant source de rayons X est, considéré dans la direction radiale, disposé davantage vers l'extérieur autour de la partie (2) d'examen que l'anneau (6) formant détecteur, dans lequel l'anneau (6) formant détecteur est disposé décalé dans la direction axiale par rapport à l'anneau (8) formant source de rayons X, de manière à ce qu'une ouverture (9) de sortie du faisceau de l'anneau (8) formant source de rayons X ne soit pas, considéré dans la direction radiale, vers la partie (2) d'examen recouverte au moins en partie de l'anneau (6) formant détecteur,
**caractérisé en ce que**
l'anneau formant détecteur comprend au moins un segment (61) de boîtier de forme annulaire ou de forme partiellement annulaire et une ou plusieurs unités (62) fonctionnelles ayant des éléments (63) de détecteur, dans lequel le segment (61) de boîtier est conformé, de manière à ce qu'au moins l'une des unités (62) fonctionnelles puisse être insérée au moins en partie dans le segment (61) de boîtier, et **en ce que**
l'anneau (6) formant détecteur comprend une première partie (11) de diaphragme, qui est fixée à l'anneau (6) formant détecteur ou qui fait partie intégralement de l'anneau (6) détecteur, dans lequel la première partie (11) de diaphragme est conformée pour donner, ensemble avec une deuxième partie (12) de diaphragme, un diaphragme (10) pour l'anneau (8) formant source de rayons X.

2. Système de tomodensitométrie assisté par ordinateur suivant la revendication 1,
dans lequel l'anneau (6) formant détecteur et l'anneau (8) formant source de rayonnement X sont montés, considéré dans la direction axiale, par des côtés différents sur le portique (4).

3. Système de tomodensitométrie assisté par ordinateur suivant la revendication 1 ou 2,
dans lequel le portique (4) entoure le diaphragme (10) de forme annulaire ayant au moins la première partie (11) de diaphragme et la deuxième partie (12) de diaphragme,
dans lequel le diaphragme est disposé devant l'ouverture (9) de sortie du faisceau de l'anneau (8) formant source de rayons X.

4. Système de tomodensitométrie assisté par ordinateur suivant la revendication 3,
dans lequel l'anneau (6) formant détecteur est conformé, de manière à pouvoir être décalé dans la direction axiale ensemble avec la première partie (11) de diaphragme, de sorte que, par un décalage de l'anneau (6) formant détecteur dans la direction axiale, une ouverture du diaphragme (10) est réglable.

5. Système de tomodensitométrie assisté par ordinateur suivant la revendication 3 ou 4,
dans lequel la deuxième partie (12) de diaphragme est montée sur l'anneau (8) formant source de rayons X ou sur une autre partie du portique (4) ou en fait intégralement partie respectivement.

6. Système de tomodensitométrie assisté par ordinateur suivant l'une des revendications 1 à 5,
dans lequel l'anneau (6) formant détecteur et l'anneau (8) formant source de rayonnement X se chevauchent en partie dans la direction axiale.

7. Système de tomodensitométrie assisté par ordinateur suivant l'une des revendications 1 à 6,
dans lequel le système de tomodensitométrie assisté par ordinateur comprend en outre une couchette (3) de patient, qui peut être entrée dans le portique (4) et en être sortie, en particulier dans la direction axiale,
dans lequel l'anneau (6) formant détecteur est monté sur le portique (4) dans la direction axiale du côté de la couchette (3) du patient.

8. Système de tomodensitométrie assisté par ordinateur suivant l'une des revendications 1 à 7,
dans lequel les unités (62) fonctionnelles peuvent être prélevées individuellement du portique (4).

9. Système de tomodensitométrie assisté par ordinateur suivant la revendication 8,
dans lequel au moins l'une des unités (62) fonctionnelles comprend un guidage (64), qui est agencé pour rendre possible une insertion précise localement de l'unité (62) fonctionnelle dans le segment (61) de boîtier.

10. Système de tomodensitométrie assisté par ordinateur suivant la revendication 8 ou 9,
dans lequel le segment (61) de boîtier comprend un collimateur (65) de rayons de dispersion, qui est agencé pour protéger des éléments (63) de détecteur de l'anneau (6) formant détecteur de rayons de dispersion de l'anneau (8) formant source de rayons X,
dans lequel le segment (61) de boîtier est conformé, de manière à ce que, lors de l'insertion de la au moins une unité (62) fonctionnelle dans le segment (61) de boîtier, les éléments (63) de détecteur de la au moins une unité (62) fonctionnelle soient insérés avec ajustement précis par rapport au collimateur de rayons de dispersion.

11. Système de tomodensitométrie assisté par ordinateur suivant l'une des revendications 8 à 10,
dans lequel l'anneau (6) formant détecteur comprend plusieurs segments (61) de boîtier, qui définissent rassemblés la forme annulaire de l'anneau (6) formant détecteur.

12. Procédé de réglage de l'ouverture de diaphragme d'un système de tomodensitométrie assisté par ordinateur comprenant un portique (4) statique, en particulier d'un système de tomodensitométrie assisté par ordinateur suivant l'une des revendications 1 à 11,
dans lequel le portique (4) comprend un anneau (6) formant détecteur et un anneau (8) formant source de rayons X ayant une ouverture (9) de sortie du faisceau, ainsi qu'un diaphragme (10) de forme annulaire ayant au moins une première partie (11) de diaphragme et une deuxième partie (12) de diaphragme,
dans lequel le diaphragme (10) est mis devant l'ouverture (9) de sortie du faisceau de l'anneau (8) formant source de rayons X, qui a un diamètre plus grand que l'anneau (6) formant détecteur,
dans lequel l'anneau (6) formant détecteur comprend au moins un segment (61) de boîtier de forme annulaire ou de forme partiellement annulaire et une ou plusieurs unités (62) fonctionnelles ayant des éléments (63) de détecteur, dans lequel le segment (61) de boîtier est conformé, de manière à ce que, au moins l'une des unités (62) fonctionnelles puisse être insérée au moins en partie dans le segment (61) de boîtier,
dans lequel la première partie de diaphragme est fixée à l'anneau (6) formant détecteur ou fait partie intégrante de l'anneau (6) formant détecteur,
dans lequel le procédé comprend le stade suivant :
- décalage de l'anneau (6) formant détecteur et ainsi de la première partie (11) de diaphragme dans la direction axiale, de manière à modifier une distance de la première partie (11) de diaphragme à la deuxième partie (12) de diaphragme et à régler l'ouverture du diaphragme (10).
